Europäisches Patentamt

European Patent Office

Office européen des brevets

(11) Numéro de publication: **0 276 587**
**A2**

## DEMANDE DE BREVET EUROPEEN

(21) Numéro de dépôt: **87402477.1**

(22) Date de dépôt: **03.11.87**

(51) Int. Cl.4 **A61N 1/30**

(30) Priorité: **05.12.86 FR 8617036**

(43) Date de publication de la demande:
**03.08.88 Bulletin 88/31**

(84) Etats contractants désignés:
**BE CH DE GB LI LU NL**

(71) Demandeur: **Bontemps, Raymond**
**5, Avenue de la Grande Armée**
**F-75016 Paris(FR)**

(72) Inventeur: **Bontemps, Raymond**
**5, Avenue de la Grande Armée**
**F-75016 Paris(FR)**

(54) **Dispositif destiné au transfert sous cutané de substances médicinales.**

(57) La présente invention concerne un dispositif avec un générateur de courant d'onde électrique, équipé d'électrodes imprégnées de substance médicinales, que l'on applique sur la surface du corps humain pour assurer le passage de ces dites substances à travers la peau.

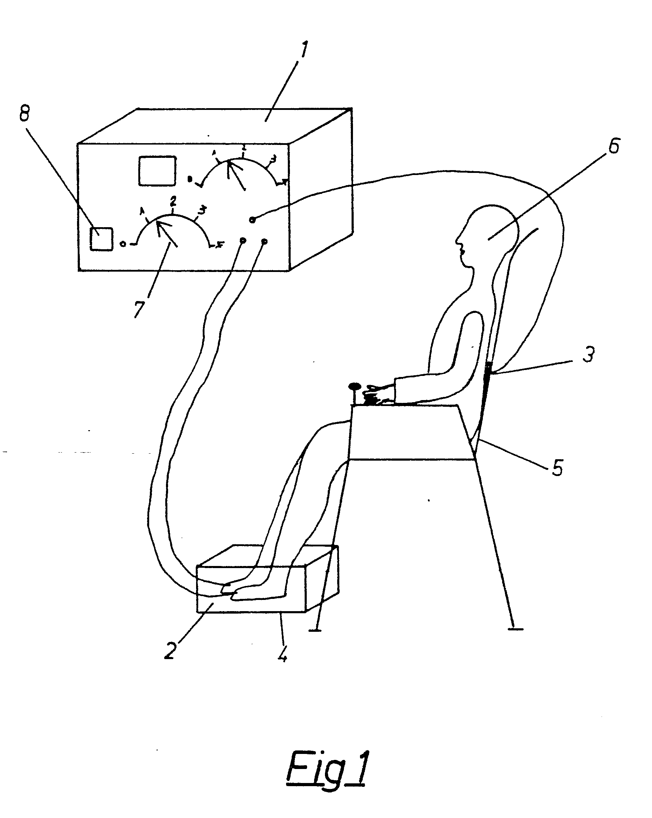

Suivant la figure 1, l'invention est caractérisée par un générateur de courant (1) à tension triangulaire fonctionnant en basse fréquence, d'électrodes de grandes surfaces (2) (3) épousant la forme d'une partie du corps, d'une réserve liquide de substance médicinale régénératrice (4) et d'un circuit de sécurité.

Le dispositif est utilisé dans le domaine de la cosmétologie et pour des applications paramédicales.

*Fig.1*

## Dispositif destiné au transfert sous-cutané de substances médicinales.

La présente invention concerne un dispositif comprenant notamment un générateur de tension fournissant deux courants dont les tensions en fonction du temps ont des amplitudes triangulaires. Ce dispositif électronique est équipé d'électrodes de grande surface que l'on applique par paire sur deux parties distinctes de la peau du corps humain. Ces électrodes imbibées de substance médicinales ou cosmétologiques elles-même conductrices d'électricité et soumises au champ électrique du générateur de tension, assurent le transfert desdites substances au travers de la peau. On connait actuellement différentes techniques destinées à provoquer le transfert sous la peau de différentes substances médicinales ou cosmétologiques.

Les techniques généralement utilisées consistent à appliquer ces substances médicinales sous forme de pommade, sur une peau préalablement dégraissée. La zone d'application est ensuite soumise à un massage afin d'assurer la pénétration de la substance.

On peut aussi employer des substances médicinales ou cosmétologiques qui sont congelées sous forme de "stick". Ces stick congelés sont directement appliqués sur la peau et la substance pénètre par effet cryothérapique. Ce dernier provoque une vasoconstriction suivie d'une dilatation des pores de la peau, observée au moment du réchauffement de celle-çi. C'est au moment de réchauffement que la substance est adsorbée.

Citons enfin, dans le cadre de traitements purement médicaux, et lorsque l'on désire que les produits pénètrent à l'intérieur du corps, l'utilisation de l'absorption par voie sublinguale, ou encore, les piqûres par voie intramusculaire.

Toutes ces techniques présentent de nombreux inconvénients et notamment:

-une faible efficacité

-l'emploi de produits concentrés en matière active

-une action non localisée

-une mauvaise spécificité.

Le dispositif électronique destiné au transfert sous-cutané de substance médicinale eprmet de résoudre ces inconvénients et notamment en:

-augmentant l'efficacité du traitement

-localisant sur le système nerveux l'action des substances médicinales

-associant à la thérapeutique appliquée une action électrique relaxante pour le patient

-augmentant la fréquence et la durée du traitement sans danger pour le patient

-banalisant l'emploi du dispositif objet de l'invention, qui peut être utilisé directement par le patient sans intervention de spécialisté médical.

Le dispositif électronique destiné au transfert sous-cutané de substance médicinale a été construit à partir des observations suivantes:

Lorsque l'on applique sur les deux faces d'une membrane échangeuse d'ions baignant dans un électrolyte et simulant le fonctionnement d'une peau humaine, un champ électrique, on provoque la migration d'ions spécifiques au travers de cette membrane vers le compactement cathodique.

Lorsque l'on applique ces mêmes électrodes deux par deux, polarisées, sur deux parties du corps humain, un couple, par exemple, placé sous la plante des pieds baignant dans un électrolyte chargé d'une substance médicinale, les autres appliquées au niveau du thorax ou des reins, on provoque la migration de la substance médicinale vers le système nerveux central.

La migration est obtenue lorsque les électrodes sont soumises au champ électrique d'un générateur de tension.

Pour éviter d'appliquer un champ électrique continu pouvant entrainer des névroses, on utilise une onde apolarisée, c'est-à-dire ayant le même signe électrique. Celle-çi, du type à balayage en dents de scie, assure un passage de courant modulé provoquant une stimulation cellulaire vasodilatatrice.

Les électrodes placées sous la plante du pied ont un temps de montée de 2,64 secondes, et fournissent un courant dont l'intensité moyenne est de 0,5 mA . Le courant retout est de 0,08 mA.

Pour les électrodes placées sur le thorax ou les reins, la montée en tension dure 13,5 secondes pour des intensités identiques.

L'impédance interne calculée est de 15 KΩ pour les courants de pied, de 20 KΩ pour les courants débités par les électrodes placées sur le thorax ou les reins. La tension fournie par les électrodes placées sous les pieds impose une tension de -25 volts à -50 volts, tandsi que les électrodes fixées au niveau du thorax ou des reins voient leur tension varier de -12,5 volts à -24 volts.

Ces variations étant commandées par le générateur de tension qui ramène, après chaque cycle, le potentiel à zéro.

La valeur de la tension, l'intensité du courant et la vitesse de balayage peuvent être réglées en fonction de la nature des substances médicinales choisies, des caractéristiques physiologiques du patient et de la durée du traitement.

A partir de ces expérimentations, on a construit le dsipositif destiné au transfert sous-cutané de substances médicinales, objet de l'invention, caractérisé en ce qu'il comporte un générateur de tension triangulaire avec un boitier alimenté à partir du secteur avec deux transformateurs, des ponts à diodes, un potentiomètre de réglage et des circuits d'isolement transistorisés, 2 moteurs des électrodes de grande surface, un contrôle tension et balayage, une réserve d'électrolyte contenant la substance médicinale, un rupteur de sécurité.

La présente invention sera mieux comprise grâce aux dessins annexés qui ne sont présentés qu'à titre indicatif et non limitatif d'une réalisation préférentielle choisie par l'inventeur.

La figure 1 représente l'organisation générale de l'invention.

la figure 2 représente, vu de l'extérieur, le générateur de tension triangulaire.

La figure 3 représente le schéma électronique du générateur de tension.

En se reportant à la figure 1 et suivant une caractéristique de l'invention, on trouve le générateur de tension triangulaire (1) placé à proximité du patient (6) et alimenté par pile ou par le secteur électrique. Ce générateur de tension alimente deux couples d'électrodes (2) et (3) par deux prises JACKS. L'un de ces couples, soutenant la plante du pied, est placé dans un récipient (4) contenant la substance médicinale, et l'autre couple (3), collé sur les reins du patient, voit l'une de ses électrodes imbibée de cette même substance.

Le patient (6) est assis sur une chaise (5), ses pieds reposent sur les électrodes (2) immergées dans le récipient (4) contenant la substance médicinale, et sur son dos, au niveau des reins, se trouve le couple d'électrodes (3) polarisé également par le générateur de tension.

Le patient dispose sur les avant-bras de la chaise d'un interrupteur de secours (9) qui arrête le champ électrique instantannément lorsque cet interrupteur n'est pas maintenu dans la position verticale, la position de repos étant toujours une position "d'arrêt" de fonctionnement du générateur électrique.

En se reportant au générateur de tension (1), ce dernier dispose d'un potentiomètre de réglage (7) de l'amplitude du balayage, et d'un interrupteur de fonctionnement (5).

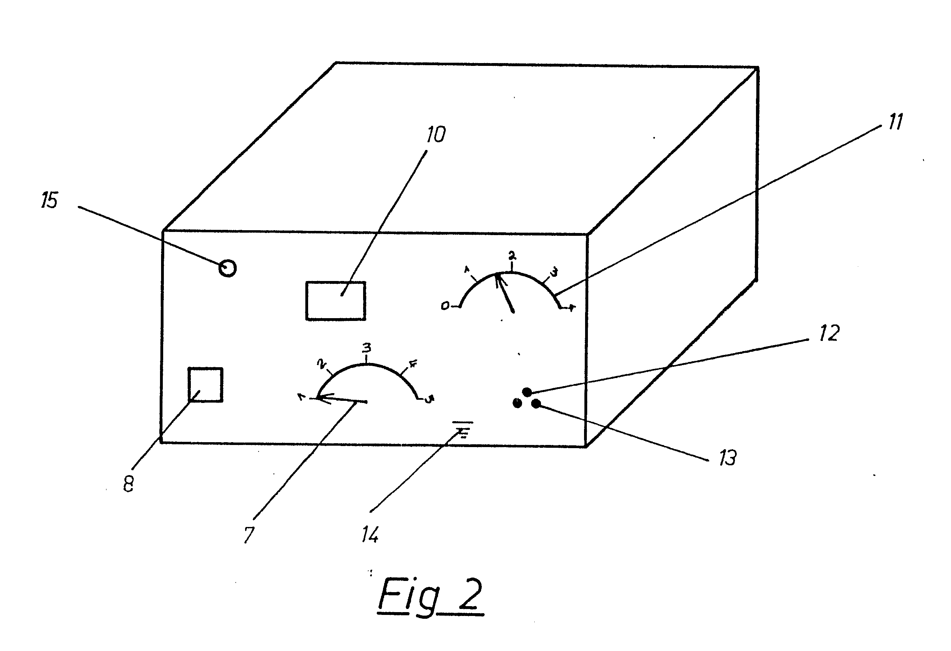

Sur la figure 2 et suivant une caractéristique importante de l'invention, le générateur d'impulsions triangulaire est présenté sur sa face avant externe.

Il est constitué d'un coffre (1) contenant l'électronique et dont le boitier est connecté à la terre au niveau (14).

Le potentiomètre (7) permet d'imposer une amplitude de tension de balayage aux signaux triangulaires qui sont générés.

Le potentiomètre (11) est destiné à régler la vitesse de balayage des signaux. reçus par les électrodes.

Le voltmètre (10) permet de visualiser l'amplitude de tension et la vitesse de balayage.

Les prises JACKS (12) et (13) constituent les sorties des signaux, le plot (12) étant relié aux électrodes placées dans les reins du patient, le plot (13) étant destiné à connecter les électrodes placées sous les pieds du patient.

L'interrupteur (8) représente l'interrupteur général du générateur et le fusible (15) est chargé d'arrêter ce générateur lorsque l'intensité du courant débité dépasse de façon continue 0,5 mA, ou que la tension excède 50 volts.

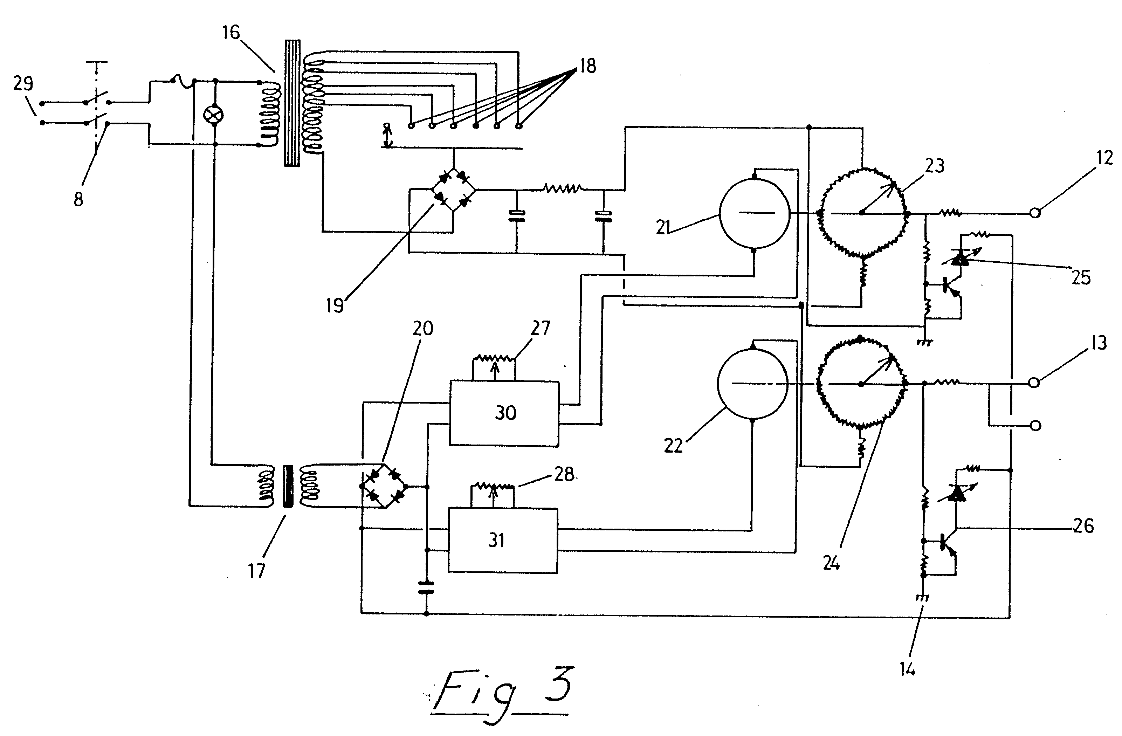

Suivant une caractéristique importante de l'invention, on a représenté sur la figure 3 l'ensemble électromécanique de commande des signaux électriques du générateur triangulaire de tension. Cet ensemble électromécanique peut facilement être remplacé par des circuits imprimés, éliminant la commande mécanique.

En se reportant à la figure 3 le générateur de tension est alimenté en courant alternatif du secteur au niveau (29). Les relais (8) sont destinés à disjoncter l'électronique lorsque le courant débité dépasse 0,5 mA.

Le transformateur (16) ajuste la tension de crête en fonction des plots (18). Le courant issu des plots (18) est redressé par le pont à diodes (19) avant d'être appliqué aux diverses électrodes (12) et (13) par l'intermédiaire des potentiomètres variables (23) et (24). La valeur de ces potentiomètres permet d'ajuster la valeur du courant débité pour chaque électrode.

Les potentiomètres variables (23) et (24) sont actionnés pra les moteurs (21) et (22) durant toute la durée de l'opération et suivant des cycles de balayage préalablement définis.

Ces moteurs (21) et (22) sont reliés à leur alimentation respective (30) et (31) que l'on règle au moyen des potentiomètres variables (27) et (28).

Les alimentations (30) et (31) sont connectées au courant secteur par l'intermédiaire du pont à diode (21) qui redresse le courant issu du transformateur (17) directement branché sur le secteur.

Le champ électrique appliqué aux électrodes est isolé de la terre par un circuit porte transistors (25) et (26) constitué des résistances R6, R7, R10, R11, des diodes, et des transistors T1 et T2.

La tension appliquée aux électrodes dépend du choix des plots réalisé au niveau (18), la vitesse de balayage est réglée par la rotation du moteur au niveau des alimentations respectives (27) et (28).

## Revendications

1 - Dispositif destiné au transfert sous-cutané de substance médicinale caractérisé en ce qu'il comporte un générateur de tension triangulaire autonome, alimenté à partir de batterie d'accumulateur ou des secteurs, incluant dans ce cas des transformateurs, pont à diode, potentiomètres de réglage, circuit d'isolement, moteur avec toujours des électrodes de grande surface, un contrôleur de tension et de balayage, des réserves d'électrolyte et des rupteurs de courant.

2 - Dispositif selon la revendication 1 caractérisé en ce que la pénétration des substances médicinales sous la peau est obtenue au moyen d'électrodes placées sous les pieds (2) et sur une autre partie haute du corps humain (3), imbibées de solution contenant la substance médicinale (4) rendue conductrice d'électricité.

3 - Dispositif selon la revendication 1 caractérisé en ce que le champ électrique appliqué aux électrodes est obtenu au moyen d'un générateur de tension triangulaire (1) fournissant une double tension variant dans le temps suivant une courbe triangulaire.

4 - Dispositif suivant la revendication 1 caractérisé en ce que le générateur de tension émet deux signaux électriques de forme triangulaire au moyen de deux moteurs (21) et (22) commandant deux potentiomètres variables (23) et (24) reliant le circuit électrique continu aux électrodes (2) et (3).

5 - Dispositif suivant la revendication 1 caractérisé en ce que la vitesse d'application des signaux électriques issus du générateur est obtenue au moyen des moteurs (21) et (22) dont les alimentations respectives (30) et (31) sont réglées par deux potentiomètres variables (27) et (28) qui commandent la vitesse desdits moteurs.

6 - Dispositif suivant la revendication 1 caractérisé en ce que la tension électrique maximale appliquée aux électrodes est réglée au moyen des tensions issues du transformateur (16) arrivant au plot (18) avant d'être redressées par le pont à diode (19) et appliquées aux électrodes

7 - Dispositif suivant la revendication 1 caractérisé en ce que le circuit de sécurité coupant le champ électrique est obtenu au moyen d'un rupteur (9) maintenant le passage du courant sous la pression manuelle du patient, et d'un relai disjonctant le générateur lorsque le courant excède une certaine valeur, la position de repos du rupteur étant celle de l'interruption du courant électrique.

8 - Dispositif selon la revendication 1 caractérisé en ce que le moyen essentiel d'assurer le transfert des produits médicinaux jusqu'aux centres nerveux du corps humain est constitué par des électrodes (2) placées sous les pieds et baignant dans une solution desdits produits médicinaux (4).

0 276 587

_Fig 1_

_Fig 2_

0 276 587

Fig 3